# EUROPEAN PATENT APPLICATION

(11) **EP 4 015 620 A1**
(43) Date of publication of application: **22.06.2022**
(21) Application number: 20852952.9
(22) Date of filing: 13.08.2020
(51) Int. Cl.: C12M 1/38, C12M 1/34, C12Q 1/686

(54) **RAPID PCR REACTION TESTING SYSTEM, AND TESTING METHOD**

(30) Priority: 13.08.2019 CN 201910742089
(71) Applicant: Girm Biosafety Technology Co. Ltd., Guangzhou, Guangdong 510530 (CN)
(72) Inventor: ZHOU, Rong, Guangzhou, Guangdong 510530 (CN); LIU, Wenkuan, Guangzhou, Guangdong 510530 (CN); LI, Xiao, Guangzhou, Guangdong 510530 (CN); WANG, Xianhua, Guangzhou, Guangdong 510530 (CN); XU, Hui, Guangzhou, Guangdong 510530 (CN); ZHOU, Zhichao, Guangzhou, Guangdong 510530 (CN); GAO, Wenjuan, Guangzhou, Guangdong 510530 (CN); LI, Lei, Guangzhou, Guangdong 510530 (CN); LIAO, Xiaohong, Guangzhou, Guangdong 510530 (CN)
(74) Representative: Petraz, Gilberto Luigi
(86) International application number: PCT/CN2020/108969
(87) International publication number: WO 2021/027891

(57) **Abstract**

The present invention discloses an ultra-rapid PCR detection system, which comprises a temperature control device, a transmission device and a reaction tube fixing device; the temperature control device at least comprises two temperature control modules, wherein at least one of the temperature control modules is a high-temperature module and at least one of the temperature control modules is a low-temperature module; a heating temperature of the high-temperature module is a first preset temperature, and a heating temperature of the low-temperature module is a second preset temperature; the transmission device is in cooperation with the temperature control device and the reaction tube fixing device, so that a reaction tube on the reaction tube fixing device is switched between the high-temperature module and the low-temperature module; the device has a compact structure and the temperature setting of the high-temperature module and the low-temperature module can promote the rising and falling rate of the temperature of the reaction mixture in the reaction tube, in addition, the rapid fluorescence PCR detection system of the present invention has good repeatability of detection results and accurate results.

## Description

### TECHNICAL FIELD

The present invention relates to a PCR detection system and a detection method, in particular an ultra-rapid PCR detection system and a detection method.

### BACKGROUND

Polymerase Chain Reaction (PCR) is an important research tool for molecular biology. Reaction time is always a limiting factor for PCR with a conventional PCR usually taking one to two hours, which limits its application in special clinical practice.

Chinese Patent Application No. CN201780033562.8 discloses a device for thermal treatment on nucleic acids according to a thermal curve for a rapid thermal cycling of sample analysis and processing. The device enables the relative reciprocating motion between a holder and at least one bath while a reactor is placed in the at least one bath by a reciprocating motor, and improves thermal conduction between a bath medium and the reactor by vibration, thereby increasing the speed of thermal cycling.

However, the device is structurally complex and requires periodic replacement of the bath medium that may also overflow from the bath. Further, the temperature setting of the bath in the device cannot meet the requirement of ultra-rapid PCR on the temperature rising and falling rate. Therefore, how to increase the temperature rising and falling rate becomes an urgent problem to be solved by an ultra-rapid PCR instrument.

### SUMMARY

In order to solve the above problems, provided is an ultra-rapid PCR detection system comprising a temperature control device, a transmission device and a reaction tube fixing device; the temperature control device at least comprises two temperature control modules, wherein at least one of the temperature control modules is a high-temperature module and at least one of the temperature control modules is a low-temperature module; a heating temperature of the high-temperature module is a first preset temperature, a heating temperature of the low-temperature module is a second preset temperature, and the first preset temperature is higher than the second preset temperature; the transmission device is in cooperation with the temperature control device and the reaction tube fixing device, so that a reaction tube on the reaction tube fixing device is switched between the high-temperature module and the low-temperature module.

Preferably, the transmission device is connected with the temperature control device and can drive the high-temperature module and the low-temperature module to move, so that the reaction tube on the reaction tube fixing device is switched between the high-temperature module and the low-temperature module.

Alternatively, the transmission device is connected with the reaction tube fixing device and drives the reaction tube fixing device to move, so that the reaction tube on the reaction tube fixing device is switched between the high-temperature module and the low-temperature module.

Alternatively, the transmission device comprises a first transmission mechanism and a second transmission mechanism, the first transmission mechanism is connected with the temperature control device and can drive the temperature control device to move, and the second transmission mechanism is connected with the reaction tube fixing device and can drive the reaction tube fixing device to move; the first transmission mechanism is in cooperation with the second transmission mechanism, so that the reaction tube on the reaction tube fixing device is switched between the high-temperature module and the low-temperature module.

Preferably, the transmission device comprises a first transmission mechanism and a second transmission mechanism, the first transmission mechanism is connected with the temperature control device and can drive the temperature control device to move horizontally, and the second transmission mechanism is connected with the reaction tube fixing device and can drive the reaction tube fixing device to move vertically; the reaction tube fixing device is disposed above the temperature control device, and the first transmission mechanism is in cooperation with the second transmission mechanism, so that the reaction tube on the reaction tube fixing device is switched between the high-temperature module and the low-temperature module.

Preferably, a lower part of the temperature control device is connected with the first transmission mechanism, and the temperature control device moves back and forth under the action of the first transmission mechanism.

Preferably, the first transmission mechanism comprises a screw motor and two guide transmission shafts, wherein the two guide transmission shafts are fixed at the front part of the screw motor, the temperature control module can move back and forth along the guide transmission shafts, and the guide rail is arranged to keep the temperature control module balanced and move stably.

Alternatively, the second transmission mechanism drives the reaction tube fixing device to move up and down through the rotation of an eccentric wheel.

Preferably, the reaction tube fixing device further comprises a transverse supporting plate fixedly mounted on a lower part of the reaction tube fixing device, the second transmission mechanism comprises a stepping motor II and an eccentric wheel, the eccentric wheel is fixed on the stepping motor II, the eccentric wheel supports the transverse supporting plate located thereon, the stepping motor II drives the eccentric wheel to rotate, and an up-and-down movement of the reaction tube fixing device is achieved by driving the transverse supporting plate to move up and down.

Preferably, the reaction tube fixing device further comprises a vertical supporting rod and a stand column, wherein the vertical supporting rod is fixed at two sides of a reaction tube fixing position, the stand column passes through the vertical supporting rod vertically, the stand column is slidably connected with the vertical supporting rod, and a spring is sleeved at the top of the stand column and abuts against the vertical supporting rod. The stand column and the vertical supporting rod are configured for limiting the direction of the up-and-down movement of the reaction tube fixing device, so the up-and-down movement of the reaction tube fixing device in a vertical direction can be ensured.

Preferably, the ultra-rapid PCR detection system further comprises a control system which is a control circuit and performs real-time control and signal transmission on the temperature control device, the first transmission mechanism, an optical detection device and the reaction tube fixing device.

Preferably, the reaction detection system further comprises an optical detection device which is positioned in front of the temperature control device.

Preferably, the optical detection device comprises an optical reading head and a third transmission mechanism, wherein the optical reading head is mounted on an upper part of the third transmission mechanism and is driven by the third transmission mechanism to move left and right.

Preferably, the third transmission mechanism comprises a stepping motor I, a guide rail, a belt and a connecting member, wherein the optical reading head is fixed at an upper part of the connecting member, a middle part of the connecting member is provided with a groove slidably connected with the guide rail, and a lower part of the connecting member is provided with a clamping plate connected with the belt.

Preferably, the reaction tube fixing device comprises a reaction tube fixing position and a gland, wherein the gland is configured for covering the reaction tube fixing position.

Preferably, the gland is made of stainless steel, and non-deformable stainless steel can produce the same gland force to sample holes at different positions, can make the reaction tubes at different positions and the temperature control module fit well to ensure the reaction tubes to be heated uniformly, and can also make the positions of the systems in the reaction tubes relative to detection holes more uniform, thereby achieving good repeatability and consistency of detection results.

Preferably, the first preset temperature is greater than or equal to 100 °C, and the second preset temperature is less than or equal to 55 °C.

Preferably, the first preset temperature is in a range of 100 to 150 °C, and the second preset temperature is in a range of 15 to 55 °C.

Preferably, the first preset temperature is 120 °C, and the second preset temperature is 47 °C.

Preferably, the temperature control device further comprises a medium-temperature module, and a heating temperature range of the medium-temperature module is between the first preset temperature and the second preset temperature.

Preferably, the temperature control module sequentially comprises a medium-temperature module, a low-temperature module and a high-temperature module from front to back, wherein a side wall of the foremost medium-temperature module is provided with detection holes, and upper parts of the medium-temperature module, the low-temperature module and the high-temperature module are all provided with sample holes.

Preferably, the temperature control module can keep the temperature constant or change the temperature.

Provided is an ultra-rapid PCR detection method, which uses the ultra-rapid PCR detection system as described above and comprises the following operation steps:
(1) preparing: starting an instrument for preheating, and filling a sample to be tested and a reagent into a reaction tube and mixing;
(2) fixedly arranging the reaction tube on a reaction tube fixing device, and starting a transmission device to ensure that the reaction tube is inserted into a high-temperature module to perform a high-temperature warm bath;
(3) starting the transmission device after the temperature of the reaction tube reaches the required temperature to ensure that the reaction tube is inserted into a low-temperature module for cooling;
(4) acquiring data; and
(5) repeating the steps (2) to (4).

Preferably, the transmission device comprises a second transmission mechanism; wherein, step (2) further comprises: moving the high-temperature module to a position right below the reaction tube, lowering the second transmission mechanism of a vertical transmission mechanism to the lowest position, and performing a high-temperature warm bath in the reaction tube; step (3) further comprises: after the temperature of the reaction tube reaches the required temperature, resetting the second transmission mechanism of the vertical transmission mechanism, moving the low-temperature module to the position right below the reaction tube, and lowering the second transmission mechanism of the vertical transmission mechanism to the lowest position for cooling.

Alternatively, or in addition, after the temperature of the reaction tube is reduced in step (3), step (3) further comprises: resetting the second transmission mechanism, and moving the medium-temperature module to the position right below the reaction tube for a certain time.

### Working Principle

The rapid PCR detection system of the present invention comprises a high-temperature module, a low-temperature module, a transmission device, an optical detection device and a reaction tube fixing device. The reaction tube can be arranged on the reaction tube fixing device and pressed by a gland after being arranged, and the reaction tube can move along with the reaction tube fixing device in the experiment. The high-temperature module provides the temperature control that exceeds a first preset temperature, and can heat the reaction tube rapidly up to a target temperature; the low-temperature module provides the temperature control of a second preset temperature, and can cool the reaction tube rapidly to a target temperature. The denaturation and annealing processes in a conventional amplification cycle adopt bypass-type temperature control, requires to keep a flow at a certain temperature for a certain time for pre-denaturation, and keeps at a continuous temperature by moving up and down at a certain frequency in the temperature control module (high-temperature module).

### Advantages

1. The reaction system moves steadily and has a compact structure, which increases the movement speed of the instrument.

The PCR tube and the temperature control device move relatively, the first transmission mechanism drives the temperature control device to move horizontally, the reaction tube fixing device is disposed above the temperature control system, and the reaction tube fixing device is connected with the second transmission mechanism and can move up and down along with the second transmission mechanism, so that the reaction tube on the reaction tube fixing device can move relatively and reciprocally between the temperature control modules; the second transmission mechanism is not connected with the first transmission mechanism, so that the first transmission mechanism cannot drive the reaction tube to move horizontally when moving; since the reaction volume for PCR is tiny, reduction of vibration of the reaction tube will prevent the reaction mixture in the reaction tube from being hung on a tube wall, avoid generation of bubbles in the reaction tube, and improve the detection accuracy.

When the instrument of the present invention is used, in order for pre-denaturation that requires keeping at a temperature for a certain period of time, the temperature control module is moved up and down at a certain frequency. In order to ensure the speed and flexibility of the up-and-down movement, the device of the present invention drives an eccentric wheel to rotate by a motor, the up-and-down movement effect can be instantly achieved only by rotating the eccentric wheel at a certain angle, and the up-and-down movement mode is faster than a belt transmission, so that the speed and efficiency of the up-and-down movement can be improved, and the accuracy of temperature control can also be improved, the movement speed of the device can be accelerated, and the detection time can be shortened.

2. The temperature setting of a high-temperature module and a low-temperature module can promote the rising and falling rate of the temperature of the reaction mixture in the reaction tube.

The temperature of a high-temperature module in a conventional PCR instrument is set up to 99 °C, while the temperature of the high-temperature module of the present invention can be set to be more than 100 °C, and multiple tests prove that the ideal heating rate can be achieved at 120 °C. Meanwhile, the detection instrument of the present invention can meet the requirements by using a common reaction tube. The set temperature of the low-temperature module of the present invention is lower than a target temperature set in the experimental process, and the low-temperature module can reach the target temperature at the maximum speed so as to improve the temperature rising and falling rate.

In the test process, when the temperature of the low-temperature module is set to be room temperature (25 °C to 30 °C), the temperature of the reaction tube is not stable in the annealing stage. The reason for the above situation is that when the temperature is lowered, the wall of the reaction tube is rapidly conducted to a lower-temperature state by the low-temperature module even if the reaction tube is separated from the low-temperature module in the later stage; the temperature of the reaction tube is lower, the low-temperature is continuously conducted from the wall of the reaction tube to an experiment system in the reaction tube after the reaction tube is separated from the low-temperature module, and the temperature lowering trend is not slowed down; when the temperature of the low-temperature module is set to be 47 °C, the reaction tube is separated from the low-temperature module after being cooled for a period of time, the annealing temperature can be stabilized, and therefore the repeatability of PCR detection can be improved in the temperature control aspect.

3. The consistency and the repeatability of detection results of different sample holes and the same sample hole are good.

Upper parts of the high-, medium- and low-temperature modules of the present invention are all provided with sample holes, a front part of the high-temperature module is provided with detection holes, and the reaction tube is tightly pressed in the sample holes through glands on the sample holes, so that the reaction tube and the temperature control module can have a good fitting degree, the temperature rising and falling rate of each sample can be improved, the temperature change consistency of each sample tube can be ensured, and the repeatability and consistency of sample detection results can be improved.

The gland of the present invention is made of hard metal, preferably stainless steel. Different gland materials can cause a great influence to detection results. When the gland is made of aluminum metal strips, due to the ductility of the glands, the press degree on top of different sample holes is different, which affects the fitting degree between the reaction tube and the temperature control module, and causes uneven heating of the reaction tube of different sample holes; meanwhile, the gland force is uneven, which can also cause the difference in the position of the system in the reaction tube relative to the detection holes, and even probably detect the interface of system and paraffin oil, finally leading to poor repeatability of the instrument. The above problem is well solved by replacing the material of the gland with stainless steel.

4. The provision of the medium-temperature module can obviously improve the amplification efficiency of PCR.

The temperature of a low-temperature pool is increased to 47 °C from room temperature, but the amplification efficiency is not improved; it can be known from observing a temperature control curve that even if the low-temperature module provides the temperature of 47 °C, but the cooling rate is too fast, and both simply providing low-temperature and separating an EP tube from the low-temperature module (tested before) cannot reduce cooling rate, so a heat source is required to heat the EP tube after annealing temperature is reached if the annealing extension temperature is required to be maintained; the preferred mode is to add a medium-temperature pool for extending temperature. The EP tube enters the low-temperature pool to reach the annealing temperature before entering the medium-temperature pool to maintain the temperature and be detected, and then enters the high-temperature pool to be subjected to denaturation and melting. The test indicated that after the medium-temperature module was added for increasing the annealing time, the time consumption for amplification efficiency was shortened, so the increase of the annealing extension time was effective for improving the amplification efficiency.

5. The rapid fluorescence PCR instrument of the present invention has no obvious difference with the common fluorescence PCR instrument in the aspects of amplification efficiency and detection limit, and has good repeatability of detection results and accurate results.

The sensitivity test of 10^1 to 10^5 copies concentration was performed on an FQPCR instrument and control QPCR instrument, the amplification efficiency and the detection limit had no obvious difference, and the two instruments could both detect a 10 copies concentration sample; it can be seen from a standard curve that the slopes of the two curves were equal, and the correlation coefficient R2 was greater than 0.99. The amplification efficiency was calculated through the standard curve, wherein the amplification efficiencies of the two instruments were close (110% to 115%).

6. According to the ultra-rapid fluorescence PCR instrument of the present invention, the temperature control module is positioned above the first transmission mechanism, and is positioned below the first transmission mechanism relative to the temperature control module, so that the sample can be placed and operated more conveniently.

7. The ultra-rapid fluorescence PCR instrument of the present invention is simple to maintain and does not need to replace a heating medium.

According to the present invention, the reaction tube is tightly pressed in the sample detection hole through the gland, which can achieve good fitting between the reaction tube and a wall surface of the sample detection hole, replaces the conventional medium heat transfer mode, reduces workload and material consumption of instrument maintenance on the one hand, can shorten the preheating time of the instrument on the other hand because the heat transfer process of a medium is omitted, and can more quickly adjust the temperature of the temperature control module.

### BRIEF DESCRIPTION OF THE DRAWINGS

FIG. 1 is a schematic configuration of an ultra-rapid PCR detection system.
FIG. 2 is a schematic configuration of a temperature control system and a first transmission mechanism.
FIG. 3 is a schematic configuration of an optical detection device.
FIG. 4 is a schematic configuration of a second transmission mechanism and a reaction tube fixing device.
FIG. 5 shows temperature test curves of a PCR instrument.
FIG. 6 shows amplification curves of different detection positions of an aluminum gland.
FIG. 7 shows amplification curves of different detection positions of a steel gland.
FIG. 8 shows amplification curves of a repeatability test at position 2.
FIG. 9 shows amplification curves of a repeatability test at position 5.
FIG. 10 shows normal distribution and histogram of Ct values.
FIG. 11 shows amplification curves (logarithmic coordinates) of sensitivity test of QPCR & FQPCR instruments.
FIG. 12 is a standard curves graph of QPCR & FQPCR instruments.

In these drawings,
1 represents a temperature control device, 11 represents a medium-temperature module, 12 represents a low-temperature module, and 13 represents a high-temperature module;
2 represents a first transmission mechanism, 21 represents a four-bar motor, and 22 represents a guide transmission shaft;
3 represents an optical detection device, 31 represents an optical reading head, 32 represents a third transmission mechanism, 321 represents a stepping motor I, 322 represents a guide rail, 323 represents a belt, 324 represents a connecting member, 3241 represents a groove, and 3242 represents a clamping plate;
4 represents a second transmission mechanism, 41 represents a stepping motor II, and 42 represents an eccentric wheel;
5 represents a reaction tube fixing device, 51 represents a transverse supporting plate, 52 represents a reaction tube fixing position, 53 represents a gland, 54 represents a vertical supporting rod, 55 represents a stand column, and 56 represents a spring; and
6 represents a control system.

The drawings in the brief description of the drawings constituting a part of the present application are used to provide a further understanding for the present invention, and the exemplary embodiments and descriptions of the present invention are provided to explain the present invention and do not constitute a limitation thereto.

In order to more clearly illustrate the technical solutions in the embodiments of the present invention, the drawings required to be used in the description of the embodiments are briefly introduced below. It is obvious that the drawings in the description below are only some embodiments of the present invention, and it is obvious for those skilled in the art that other drawings can be obtained according to the drawings without creative efforts.

### DETAILED DESCRIPTION

In order to make the objects, technical solutions and advantages of the present invention more apparent, the present invention will be further described in detail below with reference to the drawings and the detailed descriptions. It should be understood that the specific embodiments described herein are merely illustrative of the present invention and do not limit the protection scope of the present invention.

Unless defined otherwise, all technical and scientific terms used herein have the same meaning as commonly understood by those skilled in the art to which the present invention belongs. The terms used in the specification of the present invention herein are for the purpose of describing specific embodiments only and are not intended to limit the scope of the present invention. As used herein, the term "and/or" includes any and all combinations of one or more of the associated listed items.

The FQPCR described herein is an ultra-rapid fluorescence PCR instrument, and the QPCR is a common fluorescence PCR instrument.

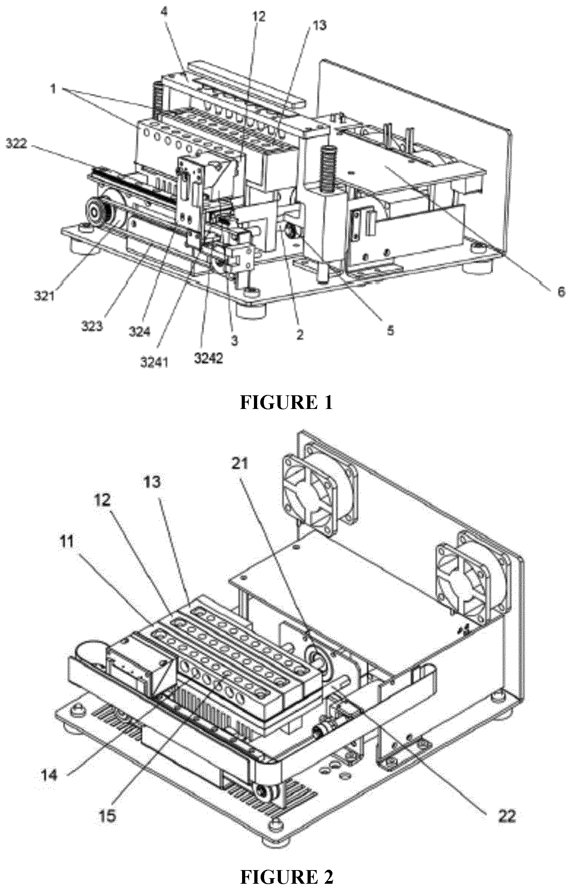

As shown in FIG. 1, an ultra-rapid PCR detection system comprises a temperature control device, a transmission device and a reaction tube fixing device.

The temperature control device at least comprises two temperature control modules, wherein at least one of the temperature control modules is a high-temperature module and at least one of the temperature control modules is a low-temperature module; a heating temperature of the high-temperature module is a first preset temperature, a heating temperature of the low-temperature module is a second preset temperature, and the first preset temperature is higher than the second preset temperature.

The transmission device is in cooperation with the temperature control device and the reaction tube fixing device, so that a reaction tube on the reaction tube fixing device is switched between the high-temperature module and the low-temperature module.

Therefore, the reaction tube can be arranged on the reaction tube fixing device and pressed by a gland after being arranged, and the reaction tube can move along with the reaction tube fixing device in the experiment. The high-temperature module provides the temperature control of the first preset temperature, and can heat the reaction tube rapidly up to a target temperature; the low-temperature module provides the temperature control of the second preset temperature, and can cool the reaction tube rapidly to the target temperature. The denaturation and annealing processes in a conventional amplification cycle adopt bypass-type temperature control, requires to keep at a certain temperature for a certain period of time for pre-denaturation, and keeps constantly at the temperature by moving up and down at a certain frequency the temperature control module (high-temperature module).

It should be noted that the minimum temperature of the first preset temperature is greater than the maximum temperature of the second preset temperature. Specific temperatures can be chosen as needed.

It should be noted that the specific implementation of the "transmission device" may be various, including but not limited to a multi-axis robot operating arm, a three-coordinate transmission device and the like, which can meet the above requirements.

Specifically, in one embodiment, the transmission device is connected with the temperature control device and can drive the high-temperature module and the low-temperature module to move, so that the reaction tube on the reaction tube fixing device is switched between the high-temperature module and the low-temperature module. Therefore, the high-temperature module and the low-temperature module of the transmission device can move to provide the required reaction temperature for the reaction tube on the reaction tube fixing device. In this case, the transmission device may be a multi-axis robot operating arm or a three-coordinate transmission device.

Alternatively, in one embodiment, the transmission device is connected with the reaction tube fixing device and drives the reaction tube fixing device to move, so that the reaction tube on the reaction tube fixing device is switched between the high-temperature module and the low-temperature module. Therefore, the reaction tube on the reaction tube fixing device can be inserted into the high-temperature module or the low-temperature module by moving, which provides the required reaction temperature for the reaction tube on the reaction tube fixing device. In this case, the transmission device may be a multi-axis robot operating arm or a three-coordinate transmission device.

Alternatively, in still another embodiment, the transmission device comprises a first transmission mechanism and a second transmission mechanism, the first transmission mechanism is connected with the temperature control device and can drive the temperature control device to move, and the second transmission mechanism is connected with the reaction tube fixing device and can drive the reaction tube fixing device to move; the first transmission mechanism is in cooperation with the second transmission mechanism, so that the reaction tube on the reaction tube fixing device is switched between the high-temperature module and the low-temperature module. Therefore, the temperature control module is controlled to move by the first transmission mechanism, so that the high-temperature module and the low-temperature module can correspond to the required reaction tubes; and then the reaction tube fixing device is driven to move by the second transmission mechanism, so that the reaction tube of the reaction tube fixing device is inserted into the required high-temperature module or low-temperature module to meet the temperature required by the PCR.

Alternatively, in yet another embodiment, the transmission device comprises a first transmission mechanism and a second transmission mechanism, the first transmission mechanism is connected with the temperature control device and can drive the temperature control device to move horizontally, and the second transmission mechanism is connected with the reaction tube fixing device and can drive the reaction tube fixing device to move vertically; the reaction tube fixing device is disposed above the temperature control device, and the first transmission mechanism is in cooperation with the second transmission mechanism, so that the reaction tube on the reaction tube fixing device is switched between the high-temperature module and the low-temperature module. The reaction tube fixing device can move up and down along with the second transmission mechanism.

The temperature control device in this embodiment comprises two temperature control modules, as shown in FIG. 1, which sequentially are a low-temperature module 12 and a high-temperature module 13 from front to back. Alternatively, the temperature control device comprises three temperature control modules, as shown in FIG. 2, which sequentially are a medium-temperature module 11, a low-temperature module 12 and a high-temperature module 13 from front to back; the temperature control module can keep the temperature constant or change the temperature.

The high-temperature module in this embodiment can set a temperature to exceed 100 °C (i.e., the first preset temperature can be set to be greater than or equal to 100 °C), making that the reaction tube can rapidly heat up to a denaturation temperature, and the low-temperature module 12 can set the temperature below the required temperature of annealing extension, making that the reaction tube rapidly cools to reach the annealing temperature, for example, below 55 °C (i.e., the second preset temperature can be set up to be less than or equal to 55 °C).

As shown in FIG. 1, a sidewall of the foremost low-temperature module is provided with detection holes 14, and upper parts of the low-temperature module 12 and the high-temperature module 13 are all provided with sample holes 15. As shown in FIG. 1, when there are three temperature control modules, a sidewall of the foremost medium-temperature module is provided with detection holes, and upper parts of the medium-temperature module 11, the low-temperature module 12 and the high-temperature module 13 are all provided with sample holes 15.

As shown in FIG. 2, the first transmission mechanism of the present invention comprises a screw motor 21 and two guide transmission shafts 22, wherein the two guide transmission shafts 22 are fixed at the front part of the screw motor 21, and the temperature control module moves back and forth along the guide transmission shafts 22.

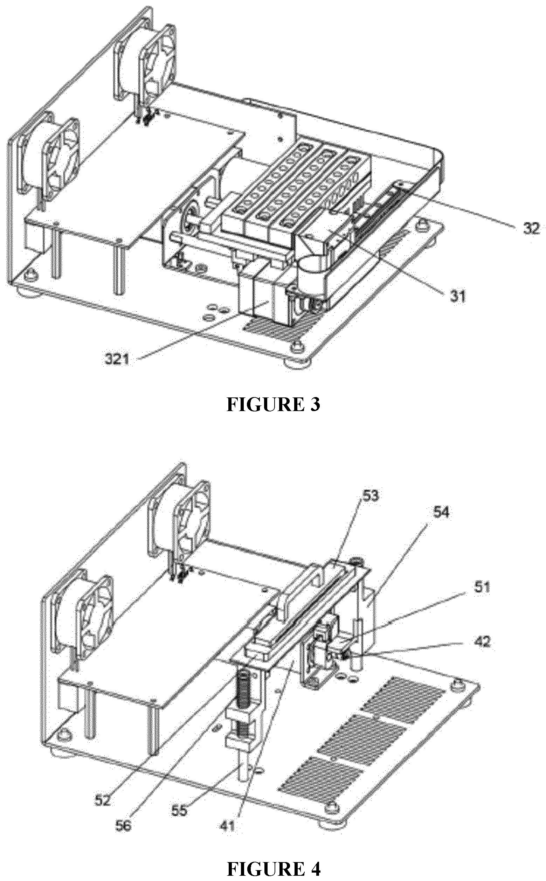

As shown in FIG. 3, the optical detection device of the present invention comprises an optical reading head 31 and a third transmission mechanism 32, wherein the optical reading head is mounted on an upper part of the third transmission mechanism and is driven by the third transmission mechanism 32 to move left and right; the third transmission mechanism 32 comprises a stepping motor I 321, a guide rail 322, a belt 323 and a connecting member 324, wherein the optical reading head 321 is fixed at an upper part of the connecting member 324, a middle part of the connecting member 324 is provided with a groove 3241 slidably connected with the guide rail, and a lower part of the connecting member is provided with a clamping plate 3242 connected with the belt.

As shown in FIG. 4, the second transmission mechanism of the present invention comprises a stepping motor II 41 and an eccentric wheel 42, the eccentric wheel 42 is fixed on the stepping motor II 41, a transverse supporting plate 51 is fixedly mounted at a lower part of the reaction tube fixing device 5, a lower part of the transverse supporting plate 51 is supported by the eccentric wheel, the stepping motor II 41 drives the eccentric wheel 42 to rotate, and an up-and-down movement of the reaction tube fixing device is achieved by driving the transverse supporting plate to move up and down; the reaction tube fixing device 5 further comprises a reaction tube fixing position 52, a gland 53, a vertical supporting rod 54 and a stand column 55, wherein the gland is arranged at the upper part of the reaction tube fixing position, the vertical supporting rod 54 is fixed at two sides of the reaction tube fixing position 52, the stand column passes through the vertical supporting rod 54 vertically, the stand column 55 is slidably connected with the vertical supporting rod 54, and a spring 56 is sleeved at the top of the stand column and abuts against the vertical supporting rod 54.

As shown in FIG. 1, the ultra-rapid PCR detection system of the present invention further comprises a control system 6, wherein a control circuit is arranged in the control system, and the control system can perform real-time control and signal transmission on the temperature control device, the first transmission mechanism, the optical detection device and the reaction tube fixing device.

In order to make the instrument to better dissipate heat, two fans are arranged above the control circuit at a rear end of the instrument of the present invention.

The reaction tube is an EP tube.

Provided is an ultra-rapid PCR detection method, which uses the ultra-rapid PCR detection system as described above and comprises the following operation steps:
(1) preparing: starting an instrument for preheating, and filling a sample to be tested and a reagent into a reaction tube and mixing;
(2) moving the high-temperature module to a position right below the reaction tube, lowering the second transmission mechanism to the lowest position, and performing a high-temperature warm bath in the reaction tube;
(3) resetting the second transmission mechanism after the temperature of the reaction tube reaches the required temperature, moving the low-temperature module to the position right below the reaction tube, and lowering the second transmission mechanism to the lowest position for cooling;
(4) resetting the second transmission mechanism, and moving the medium-temperature module to the position right below the reaction tube for a certain time;
(5) acquiring data; and
(6) repeating the steps (2) to (5).

When the above methods and devices are used to perform nucleic acid analysis and processing, the samples and reagents described above comprise reaction components including at least one enzyme, nucleic acids and/or particles containing at least one nucleic acid, primers for PCR, primers for isothermal amplification, primers for other nucleic acid amplification and processing, dNTP, Mg²⁺, fluorescent dyes and probes, control DNA, control RNA, control cells and control microorganisms, and other reagents necessary for nucleic acid amplification, processing and analysis. The particles containing nucleic acid comprise at least one cell virus, white blood cells and stroma cells, circulating tumor cells and embryo cells.

The above methods and devices are used for pre-amplification or template enrichment of polymerase chain reaction, reverse transcription-polymerase chain reaction, end-point PCR, ligase chain reaction, nucleic acid sequencing or variations of each polymerase chain reaction (PCR), isothermal amplification, linear amplification, library preparation for sequencing, and bridge amplification for sequencing. Variations of the above polymerase chain reaction comprise reverse transcription-PCR, real-time fluorescent quantitative polymerase chain amplification reaction and real-time fluorescent quantitative reverse transcription-polymerase chain amplification reaction, reverse polymerase chain amplification reaction, anchored polymerase chain amplification reaction, asymmetric polymerase chain amplification reaction, multiplex PCR, color complementary polymerase chain amplification reaction, immune polymerase chain amplification reaction, nested polymerase chain amplification reaction, template enrichment for pre-amplification or nucleic acid sequencing, and ELISA-PCR.

### TESTING EXAMPLES

### 1. The reaction system moves steadily and has a compact structure, which increases the movement speed of the instrument.

The PCR tube and the temperature control device move relatively, the first transmission mechanism drives the temperature control device to move horizontally, the reaction tube fixing device is disposed above the temperature control system, and the reaction tube fixing device is connected with the second transmission mechanism and can move up and down along with the second transmission mechanism, so that the reaction tube on the reaction tube fixing device can move relatively and reciprocally between the temperature control modules; the second transmission mechanism is not connected with the first transmission mechanism, so that the first transmission mechanism cannot drive the reaction tube to move horizontally when moving; PCR volume is smaller, so that the vibration of the reaction tube is reduced, the reaction mixture in the reaction tube can be prevented from being hung on a tube wall, the generation of bubbles in the reaction tube can be avoided, and the detection accuracy is improved.

When the instrument of the present invention is used, in order to keep at a temperature for a certain period of time for pre-denaturation, the temperature control module is moved up and down at a certain frequency. In order to ensure the speed and flexibility of the up-and-down movement, the device of the present invention drives an eccentric wheel to rotate by a motor, the up-and-down movement effect can be instantly achieved only by rotating the eccentric wheel at a certain angle, and the up-and-down movement mode is faster than a belt transmission, so that the speed and efficiency of the up-and-down movement can be improved, and the accuracy of temperature control can be improved, the movement speed of the device can be accelerated, and the detection time can be shortened.

### 2. The temperature setting of a high-temperature module and a low-temperature module can promote the rising and falling rate of the temperature of the reaction mixture in the reaction tube.

The temperature of a high-temperature module in a conventional PCR instrument is set up to 99 °C, while the temperature of the high-temperature module of the present invention is set to be more than 100 °C, and multiple tests proved that the ideal heating rate can be achieved at 120 °C, and meanwhile, the detection instrument of the present invention can meet the requirements by using a common reaction tube.

The set temperature of the low-temperature module of the present invention is lower than a target temperature set in the experimental process, and the low-temperature module can reach the target temperature at the maximum speed so as to improve the temperature rising and falling rate.

Testing conditions: the first preset temperature was 120 °C, and the second preset temperature was below 40 °C.

Setting amplification conditions:
40 cycles of
Denaturation: 88 °C, 1 sec; and
Annealing: 66 °C, for 1 sec; starting a next process after detection.

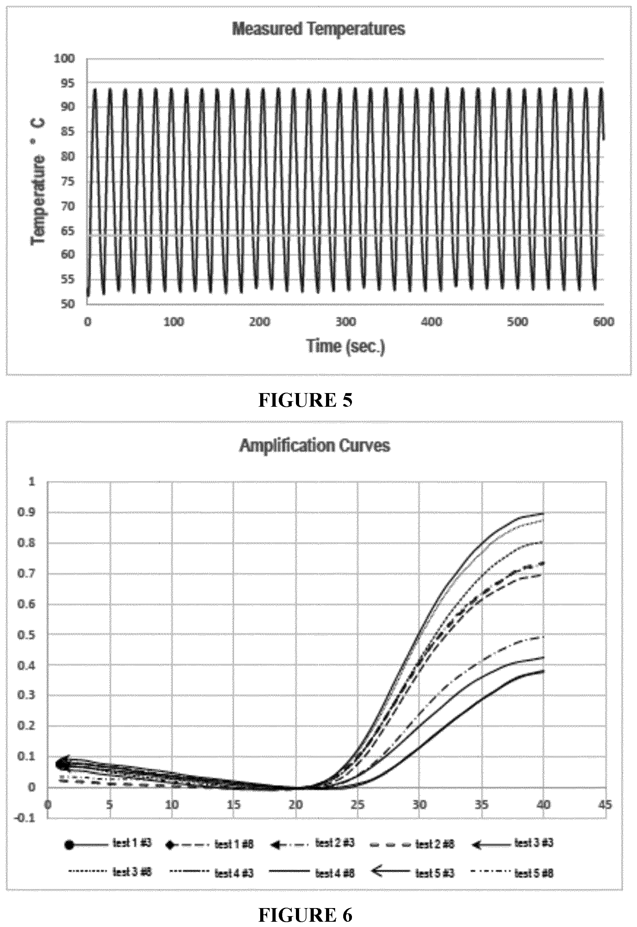

The temperature curve of the experiment was recorded, as shown in FIG. 5.

It can be known from the analysis that the maximum temperature rising speed of the temperature module was 10 °C/s, the temperature falling speed thereof was 8 °C/s, and the average temperature rising and falling rate was higher than 7 °C/s. For practical operation at 65-90 °C, temperature rising and falling was approximately 7 seconds, when pluses 1 second of melting and 1 second of extension and 1 second of detection to obtain a total of 1 cycle for 10 seconds; 40 cycles took 440 seconds which approximately equals to 6.6 minutes, then pluses a 2-minute of pre-denaturation to obtain a total of approximately 8.6 minutes for completing the PCR.

In the test process, when the temperature of the low-temperature module was set to be room temperature (25 °C to 30 °C), the temperature of the reaction tube was not stable in the annealing stage. The reason for the above situation was that when the temperature was lowered, the wall of the reaction tube was rapidly conducted to a lower-temperature state by the low-temperature module even if the reaction tube was separated from the low-temperature module in the later stage; the temperature of the reaction tube was lower, the low-temperature was continuously conducted from the wall of the reaction tube to an experiment system in the reaction tube after the reaction tube was separated from the low-temperature module, and the temperature lowering trend was not slowed down; when the temperature of the low-temperature module was set to be 47 °C, the reaction tube was separated from the low-temperature module after being cooled for a period of time, the annealing temperature can be stabilized, and therefore the repeatability of PCR detection can be improved in the temperature control aspect.

### 3. The consistency and the repeatability of detection results of detection holes of different samples are good.

Upper parts of the high-temperature module, the medium-temperature module and the low-temperature module of the present invention are all provided with sample holes, a front part of the high-temperature module is provided with detection holes, and the reaction tube is tightly pressed in the sample holes through glands on the sample holes, so that the reaction tube and the temperature control module can have a good fitting degree, the temperature rising and falling rate of each sample can be improved, the temperature change consistency of each sample tube can be ensured, and the repeatability and consistency of sample detection results can be improved.

The gland of the present invention is made of hard metal, preferably stainless steel. Different gland materials can cause a great influence to detection results. When the gland is made of aluminum metal strips, due to the easy deformation of the glands, the press degree on top of different sample holes is different, which affects the fitting degree between the reaction tube and the temperature control module, and causes uneven heating of the reaction tube of different sample holes; meanwhile, the gland force is uneven, which also can cause the difference in the position of the system in the reaction tube relative to the detection holes, and even probably detect the interface of system and paraffin oil, finally leading to the repeatability of the instrument not good. The above problem is well solved by replacing the material of the gland with stainless steel.

The specific detection process is as follows.

### Control group 1: the gland used by the instrument is made of aluminum metal

The temperature control modules were sequentially numbered as No. 1 to No. 8 from left to right, and 1 test was performed at each of 8 positions from No. 1 to No. 8 to investigate the repeatability of different hole positions. Before testing, a 10X testing system was prepared at one time and aliquoted into 8 100 µL of transparent reaction tubes, each tube was aliquoted with 20 µL of the mixed system and 20 µL of paraffin oil and then placed at positions No. 1 to No. 8 for testing, and the temperature control process as follows.

Setting amplification conditions:
40 cycles of
Pre-denaturation: 88 °C, 2 min;
Denaturation: 88 °C, 1 sec; and
Annealing: 64°C, for 1 sec; starting a next process after detection.

The detection results are shown in FIG. 6, and it can be seen in FIG. 6 that the repeatability of position No. 8 was not bad, but the repeatability of position No. 3 is poor.

### Experimental groups: the gland used by the instrument is made of stainless steel

The detection holes of the temperature control module are sequentially numbered as No. 1 to No. 8 from left to right, tests are performed at positions No. 3, No. 5 and No. 7, and the above tests are repeated for 3 times to obtain a total of 9 curves to investigate the repeatability of different hole positions.

Setting amplification conditions:
40 cycles of
Pre-denaturation: 88 °C, 2 min;
Denaturation: 88 °C, 1 sec; and
Annealing: 64°C, for 1 sec; starting a next process after detection.

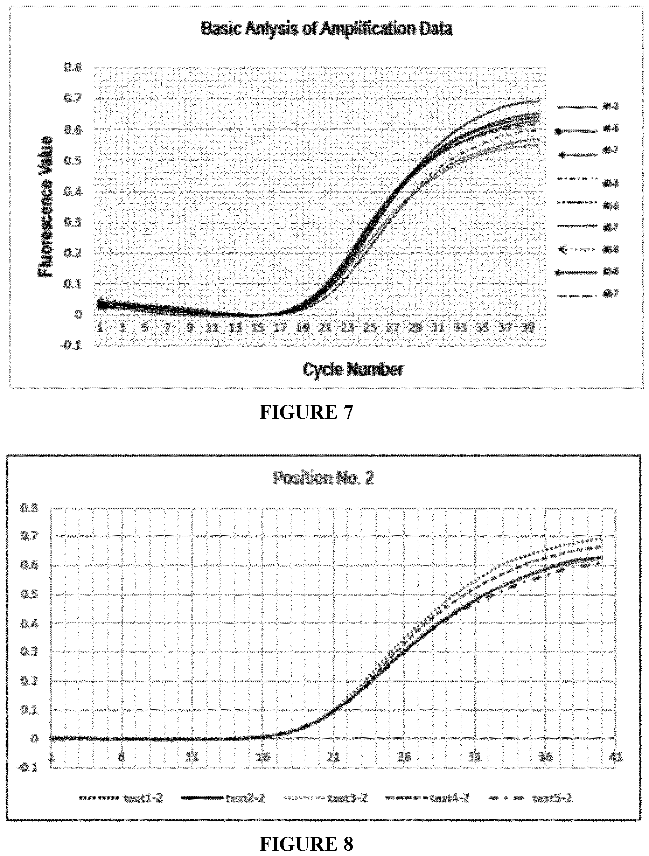

Before testing, a 10X testing system was prepared at one time and as aliquoted into 9 100 µL of transparent reaction tubes, and each tube was aliquoted with 20 µL of the mixed system and 20 µL of paraffin oil and then placed at positions No. 3, No. 5 and No. 7 for testing. The results are shown in FIG. 7.

As shown in Figures 6 and 7, the results show that gland materials had a great influence on the repeatability of detection results of different hole positions, and only when a metal which is not easy to deform was used and reaction tubes in detection holes at different positions on the temperature control module can be tightly pressed, the reaction tube and the detection module had a good fitting degree, and the repeatability can meet detection requirements.

### 4. Good repeatability in the same hole position

The specific detection process is as follows:
The temperature control modules were sequentially numbered as No. 1 to No. 8 from left to right, tests were performed at positions No. 2 and No. 5, and the above tests were repeated for 5 times to investigate the repeatability of different hole positions. Before testing, a 10X testing system was prepared at one time and aliquoted into 10 100 µL of transparent reaction tubes, and each tube was aliquoted with 20 µL of the mixed system and 20 µL of paraffin oil and then placed in a refrigerator (2°C-8°C) for storage; two reaction tubes were taken out from the refrigerator before each experiment and then placed at positions No. 2 and No. 5 for testing, and the temperature control process was as follows:

In this detection, the temperature control module of the rapid PCR instrument was set as follows: the high-temperature module was 120 °C, and the low-temperature module was 47 °C.

Setting amplification conditions:
40 cycles of
Pre-denaturation: 88 °C, 2 min;
Denaturation: 88 °C, 1 sec; and
Annealing: 64°C, for 1 sec; starting a next process after detection.

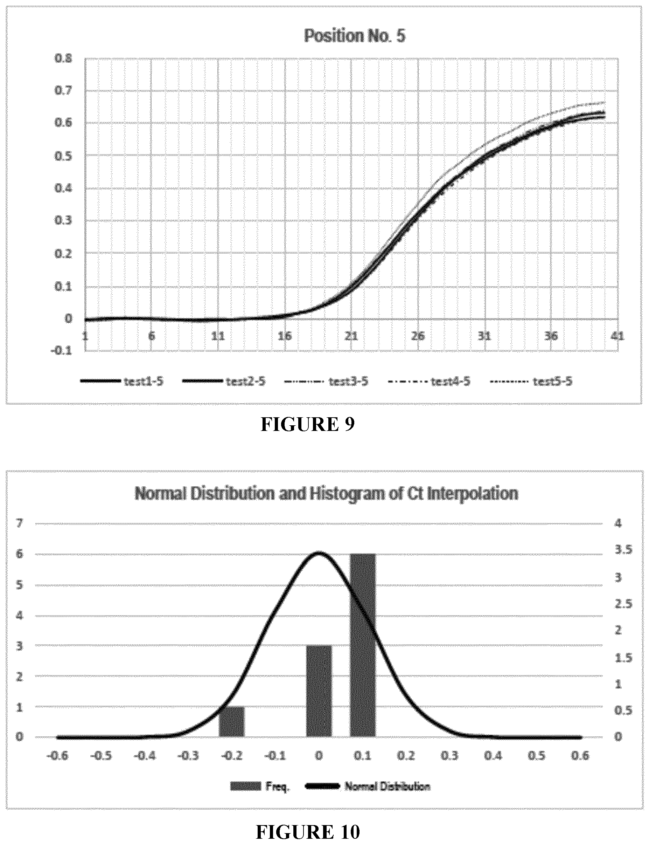

The detection results are shown in Figures 8 and 9, and it can be known from the amplification curves of 5 times of repeated tests at each position of positions No. 2 and No. 5 that single hole position test has better repeatability.

Specific test data are shown in the following table.

**Table 1. Repeatability data of Ct value of positions No. 2 and No. 5**

| Ct | Test1 | Test2 | Test3 | Test4 | Test5 | Mean Value | Range | SD |
|---|---|---|---|---|---|---|---|---|
| Position No. 2 | 19.04 | 18.96 | 19.00 | 19.02 | 18.91 | 18.99 | 0.13 | 0.0511 |
| Position No. 5 | 18.94 | 19.03 | 18.85 | 19.03 | 18.66 | 18.90 | 0.36 | 0.1519 |
| Mean Value | 18.95 | | | | | | | |
| Range | 0.38 | | | | | | | |
| SD | 0.1159 | | | | | | | |

It can be known from the analysis of Ct values in the above table that ranges of positions No. 2 and No. 5 are not more than 0.4, and the total range is not more than 0.4 when all 10 Ct values are analyzed together. Normal distribution and histogram are performed on the Ct values of the 5 repeated tests at positions No. 2 and No. 5 in the above table, and it can be seen from FIG. 10 that 9 out of 10 Ct values are within ±0.1 range from the mean value, and only 1 tested Ct value differs from the mean value by more than 0.2.

As shown in the above data, the detection instrument of the present invention has good repeatability in the same hole position.

### 5. The arrangement of the medium-temperature module can obviously improve the amplification efficiency of PCR.

The temperature of a low-temperature pool was increased to 47 °C from room temperature, but the amplification efficiency was not improved; it can be known from observing a temperature control curve that even if the low-temperature module provides the temperature of 47 °C, but the cooling rate was too fast, and both simply providing low-temperature and separating an EP tube from the low-temperature module cannot reduce cooling rate, so a heat source is required to heat the EP tube after annealing temperature is reached if the annealing extension temperature is required to be maintained; the preferred mode was to add a medium-temperature pool for extending temperature. The EP tube entered the low-temperature pool to reach the annealing temperature before entering the medium-temperature pool to maintain the temperature and be detected, and then entered the high-temperature pool to be subjected to denaturation and melting.

### 6. The rapid fluorescence PCR instrument of the present invention has no obvious difference with the common fluorescence PCR instrument in the aspects of amplification efficiency and detection limit, and has good repeatability of detection results and accurate results.

In this detection, the temperature control module of the rapid PCR instrument was set as follows: the high-temperature module was 120 °C, and the low-temperature module was 47 °C.

Sample treatment: performing copy amplification experiments with different concentration gradients of 10^1 to 10^5 by using the rapid fluorescence PCR instrument of this experiment and a standard QPCR instrument, and investigating sensitivity and a detection range;

The experimental process was as follows:
40 cycles of
Pre-denaturation: 90°C, 2 min;
Denaturation: 90°C, 1 sec; and
Annealing: 60 °C, for 7 sec; starting a next process after detection.

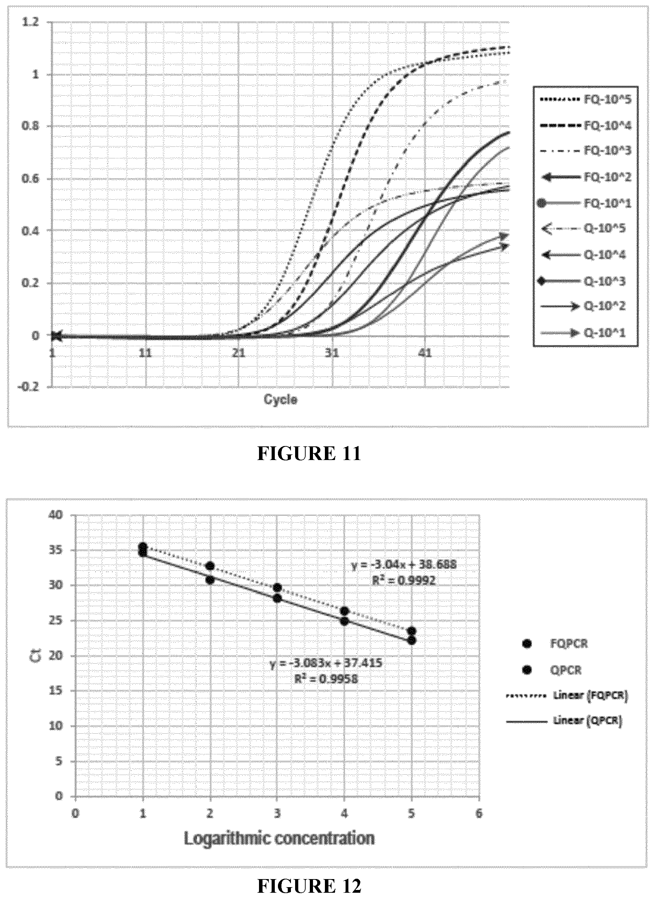

FIG. 11 shows the amplification of 10^1 to 10^5 copies using a QPCR instrument and FQPCR used for control. It can be seen from the curves that the test of the two instruments has no significant difference, and the Ct value intervals between different concentration samples are also equivalent.

**Table 2. Amplification Ct values of sensitivity tests using QPCR and FQPCR**

| Number of copies | 5 | 4 | 3 | 2 | 1 | k | E |
|---|---|---|---|---|---|---|---|
| FQPCR | 23.51 | 26.38 | 29.67 | 32.76 | 35.52 | -3.04 | 113.28% |
| QPCR | 22.2 | 24.94 | 28.21 | 30.79 | 34.69 | -3.083 | 111.01% |

Table 2 shows the amplification Ct values of sensitivity tests using QPCR and FQPCR, and FIG. 12 shows a standard curve obtained by performing sensitivity tests on the two instruments and performing gradient dilution tests on the quantitative control. It can be seen from the standard curve that the slopes of the two curves are equivalent, and the correlation coefficient R² is also greater than 0.99. The amplification efficiency is calculated through the standard curve, wherein the amplification efficiencies of the two instruments are close (110% to 115%).

The sensitivity test of 10^1 to 10^5 copies concentration is performed on an FQPCR instrument and control QPCR instrument, the amplification efficiency and the detection limit have no obvious difference, and the two instruments can both detect a 10 copies concentration sample.

The sensitivity test of 10^1 to 10^5 copies concentration is performed on an FQPCR instrument and control QPCR instrument, and the amplification efficiency and the detection limit have no obvious difference.

It should be noted that "a certain body" and "a certain part" may be a part of a corresponding "member", that is, "a certain body" and "a certain part" may be manufactured by being integrally formed with "other parts of the member"; or an independent component which can be separated from "other parts of the member", that is, "a certain body" and "a certain part" can be manufactured independently and integrally combined with "other parts of the component". The expressions "a certain body" and "a certain part" in the present application are only one embodiment for easy reading and are not intended to limit the scope of the present application, and should be construed as equivalents of the present application as long as the above features are included and the effects are the same.

It should be noted that, the components included in the "unit", "assembly", "mechanism" and "apparatus" of the present application can also be flexibly combined, that is, can be produced in a modularized manner according to actual needs, so as to facilitate modularized assembly. The division of the above components in the present application is only one embodiment for easy reading and are not intended to limit the scope of the present application, and should be construed as equivalents of the present application as long as the above components are included and the effects are the same.

In the description of the present invention, it should be understood that directions or positional relationships indicated by terms such as "central", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential" and the like are those shown based on the accompanying drawings, are merely intended to facilitate and simplify description rather than indicate or imply that the indicated device or element must have a specific direction and be structured and operated according to the specific direction, and should not be construed as limiting the present invention.

Furthermore, the terms "first" and "second" are used for descriptive purpose only rather than construed as indicating or implying relative importance or implicitly indicating the number of indicated technical features. Therefore, features defined with "first" and "second" may explicitly or implicitly include at least one of the features. In the description of the present invention, unless otherwise clearly and specifically defined, "a plurality of" means at least two, e.g., two or three.

In the present invention, unless otherwise clearly specified and defined, the terms "mount", "connect with", "connect", "fix" and the like should be comprehended in their broad sense. For example, "connect" may be "fixedly connect", "detachably connect" or "integrally connect"; "mechanically connect" and "electrically connect"; or "directly interconnect", "indirectly interconnect through an intermediate", "the communication between the interiors of two elements" or "the interaction between two elements", unless otherwise clearly defined. For those of ordinary skill in the art, the specific meanings of the aforementioned terms in the present invention can be understood according to specific conditions.

In the present invention, unless otherwise clearly specified and defined, a first feature "on" or "under" a second feature may be the first feature directly contacting with the second feature or the first and second features may be indirectly contacting with each other through an intervening intermediate. Also, a first feature "on", "above", and "over" a second feature may be the first feature directly on or obliquely above the second feature, or simply mean that the first feature is at a higher level than the second feature. A first feature "under", "beneath", and "below" a second feature may be the first feature directly under or obliquely under the second feature, or simply means that the first feature is at a lower level than the second feature.

It should be noted that, when an element is referred to as being "fixed", "disposed", "secured" or "mounted" to another element, it can be directly on the other element or can also be on the other element by an intervening element. When an element is considered as being "connected" to another element, it can be directly connected to the other element or may be connected to the other element by an intervening element. Further, when one element is considered as being "fixedly and drivingly connected" to another element, the two elements may be fixed by detachable connection or non-detachable connection, which can realize power transmission, such as sleeving, clamping, integrally-formed fixing, welding and the like; it can be realized in the prior art and is not described herein. When an element is perpendicular or approximately perpendicular to another element, it is desirable that the two elements are perpendicular, but there may be some perpendicular errors due to manufacturing and assembly effects. The terms "vertical", "horizontal", "left", "right" and the like as used herein are for illustrative purposes only and are not intended to represent only one embodiment.

Technical features in the above embodiments may be combined in any combinations. In order to make the description brief, all possible combinations of various technical features in the above embodiments are not described; however, it should be considered as being within the scope of this specification as long as there is no contradiction in the combinations of the technical features.

The above examples only illustrate several embodiments of the present invention for the purpose of specific and detailed descriptions, but should not be construed as limiting the scope of the present invention. It should be noted that various changes and modifications can be made by those skilled in the art without departing from the spirit of the present invention, and these changes and modifications are all within the scope of the present invention. Therefore, the protection scope of the present invention should be determined with reference to the appended claims.

## Claims

1. An ultra-rapid PCR detection system, comprising a temperature control device, a transmission device and a reaction tube fixing device;
the temperature control device at least comprises two temperature control modules, wherein at least one of the temperature control modules is a high-temperature module and at least one of the temperature control modules is a low-temperature module;
a heating temperature of the high-temperature module is a first preset temperature, a heating temperature of the low-temperature module is a second preset temperature, and the first preset temperature is higher than the second preset temperature;
the transmission device is in cooperation with the temperature control device and the reaction tube fixing device, so that a reaction tube on the reaction tube fixing device is switched between the high-temperature module and the low-temperature module.

2. The ultra-rapid PCR detection system according to claim 1, wherein the transmission device is connected with the temperature control device and can drive the high-temperature module and the low-temperature module to move, so that the reaction tube on the reaction tube fixing device is switched between the high-temperature module and the low-temperature module; or
alternatively, the transmission device is connected with the reaction tube fixing device and drives the reaction tube fixing device to move, so that the reaction tube on the reaction tube fixing device is switched between the high-temperature module and the low-temperature module; or
alternatively, the transmission device comprises a first transmission mechanism and a second transmission mechanism, the first transmission mechanism is connected with the temperature control device and can drive the temperature control device to move, and the second transmission mechanism is connected with the reaction tube fixing device and can drive the reaction tube fixing device to move; the first transmission mechanism is in cooperation with the second transmission mechanism, so that the reaction tube on the reaction tube fixing device is switched between the high-temperature module and the low-temperature module.

3. The ultra-rapid PCR detection system according to claim 1, wherein the transmission device comprises a first transmission mechanism and a second transmission mechanism, the first transmission mechanism is connected with the temperature control device and can drive the temperature control device to move horizontally, and the second transmission mechanism is connected with the reaction tube fixing device and can drive the reaction tube fixing device to move vertically; the reaction tube fixing device is disposed above the temperature control device, and the first transmission mechanism is in cooperation with the second transmission mechanism, so that the reaction tube on the reaction tube fixing device is switched between the high-temperature module and the low-temperature module.

4. The ultra-rapid PCR detection system according to claim 3, wherein a lower part of the temperature control device is connected with the first transmission mechanism, and the temperature control device moves back and forth under the action of the first transmission mechanism;
preferably, the first transmission mechanism comprises a screw motor and two guide transmission shafts, wherein the two guide transmission shafts are fixed at the front part of the screw motor, and the temperature control module can move back and forth along the guide transmission shafts; or
alternatively, the second transmission mechanism drives the reaction tube fixing device to move up and down through the rotation of an eccentric wheel.

5. The ultra-rapid PCR detection system according to claim 3, wherein the reaction tube fixing unit further comprises a transverse supporting plate, and the transverse supporting plate is fixedly mounted at a lower part of the reaction tube fixing device; the second transmission mechanism comprises a stepping motor II and an eccentric wheel, the eccentric wheel is fixed on the stepping motor II, the eccentric wheel supports the transverse supporting plate located thereon, the stepping motor II drives the eccentric wheel to rotate, and an up-and-down movement of the reaction tube fixing device is achieved by driving the transverse support plate to move up and down;
preferably, the reaction tube fixing device further comprises a vertical supporting rod and a stand column, wherein the vertical supporting rod is fixed at two sides of a reaction tube fixing position, the stand column passes through the vertical supporting rod vertically, the stand column is slidably connected with the vertical supporting rod, and a spring is sleeved at the top of the stand column and abuts against the vertical supporting rod.

6. The ultra-rapid PCR detection system according to claim 3, further comprising a control system, wherein the control system is a control circuit and performs real-time control and signal transmission on the temperature control device, the first transmission mechanism, an optical detection device and the reaction tube fixing device.

7. The ultra-rapid PCR detection system according to claim 1, wherein the reaction detection system further comprises an optical detection device, and the optical detection device is positioned in front of the temperature control device;
preferably, the optical detection device comprises an optical reading head and a third transmission mechanism, wherein the optical reading head is mounted on an upper part of the third transmission mechanism and is driven by the third transmission mechanism to move left and right;
preferably, the third transmission mechanism comprises a stepping motor I, a guide rail, a belt and a connecting member, wherein the optical reading head is fixed at an upper part of the connecting member, a middle part of the connecting member is provided with a groove slidably connected with the guide rail, and a lower part of the connecting member is provided with a clamping plate connected with the belt; or/and the reaction tube fixing device comprises a reaction tube fixing position and a gland, wherein the gland is configured for covering the reaction tube fixing position; and
preferably, the gland is made of stainless steel.

8. The ultra-rapid PCR detection system according to any one of claims 1 to 7, wherein the first preset temperature is greater than or equal to 100 °C, and the second preset temperature is less than or equal to 55 °C;
preferably, the first preset temperature is in a range of 100 to 150 °C, and the second preset temperature is in a range of 15 to 55 °C;
preferably, the temperature control device further comprises a medium-temperature module, and a heating temperature range of the medium-temperature module is between the first preset temperature and the second preset temperature;
preferably, the temperature control module sequentially comprises a medium-temperature module, a low-temperature module and a high-temperature module from front to back;
preferably, a side wall of the foremost medium-temperature module is provided with detection holes, and upper parts of the medium-temperature module, the low-temperature module and the high-temperature module are all provided with sample holes; and
preferably, the temperature control module can keep the temperature constant or change the temperature.

9. An ultra-rapid PCR detection method, comprising the following operation steps:
(1) preparing: starting an instrument for preheating, and filling a sample to be tested and a reagent into a reaction tube and mixing;
(2) fixedly arranging the reaction tube on a reaction tube fixing device, and starting a transmission device to ensure that the reaction tube is inserted into a high-temperature module to perform a high-temperature warm bath;
(3) starting the transmission device after the temperature of the reaction tube reaches the required temperature to ensure that the reaction tube is inserted into a low-temperature module for cooling;
(4) acquiring data; and
(5) repeating the steps (2) to (4).

10. The ultra-rapid PCR detection method according to claim 9, wherein the transmission device comprises a second transmission mechanism; wherein, step (2) further comprises: moving the high-temperature module to a position right below the reaction tube, lowering the second transmission mechanism of a vertical transmission mechanism to the lowest position, and performing a high-temperature warm bath in the reaction tube; step (3) further comprises: after the temperature of the reaction tube reaches the required temperature, resetting the second transmission mechanism of the vertical transmission mechanism, moving the low-temperature module to the position right below the reaction tube, and lowering the second transmission mechanism of the vertical transmission mechanism to the lowest position for cooling;
or/and, after the temperature of the reaction tube is reduced in step (3), step (3) further comprises: resetting the second transmission mechanism, and moving the medium-temperature module to the position right below the reaction tube.
